# EUROPÄISCHE PATENTSCHRIFT

(11) **EP 1 998 726 B1**
(45) Veröffentlichungstag und Bekanntmachung des Hinweises auf die Patenterteilung: **22.09.2010**
(21) Anmeldenummer: 07723797.2
(22) Anmeldetag: 30.03.2007
(51) Int. Cl.: A61F 5/01

(54) **ORTHESE ZUR KLUMPFUSSBEHANDLUNG**
ORTHOSIS FOR TREATMENT OF CLUBFOOT
ORTHESE POUR TRAITEMENT DES PIEDS BOTS

(30) Priorität: 30.03.2006 DE 202006005117 U
(43) Veröffentlichungstag der Anmeldung: 10.12.2008
(73) Patentinhaber: Semeda Medizinische Instrumente e.K., 22393 Hamburg (DE)
(72) Erfinder: KUJUS, Harald, 22393 Hamburg (DE); Dr.SINCLAIR, Marc, Dubai (AE)
(74) Vertreter: Hauck Patent- und Rechtsanwälte
(86) Internationale Anmeldenummer: PCT/EP2007/002855
(87) Internationale Veröffentlichungsnummer: WO 2007/112962

(56) Entgegenhaltungen:
- DE-A1- 2 749 411
- US-A- 3 477 426
- US-A- 4 040 416
- US-A- 4 157 876
- US-A- 5 346 463

## Beschreibung

Die Erfindung bezieht sich auf eine Orthese zur Klumpfußbehandlung.

Bekannt ist die Behandlung des Klumpfußes durch Gipsredressionstherapie. Der Fuß wird gerichtet und ein Gips an den gerichteten Fuß angelegt. Dies erfolgt in einigen Schritten, wobei der Fuß immer weiter in Korrekturrichtung geformt wird.

Ferner bekannt sind Orthesen zur konservativen Klumpfußbehandlung nach Ponseti. Bei einer bekannten Orthese aus starrem Kunststoff sind zwei parallele Stangen in einer Klemmvorrichtung einstellbar fixiert. Die Stangen haben an ihren äußeren Enden festklemmbare Gelenke, die mit Platten verbunden sind. Die Platten sind lösbar mit Schuhen des Patienten verbindbar. Die Gelenke ermöglichen ein Einstellen und Festlegen der Füße im geforderten therapeutischen Bereich der Dorsalextension (Anheben des Fußes zum Körper) von 0° bis 20° und der Abduktion (Schwenken der Füße nach außen) im Bereich von 0° bis 80°.

Die Therapie beginnt direkt nach der Geburt mit der Gipsredressionstherapie. Einige Wochen später wird die Orthese angewandt. Die Therapie endet etwa mit dem 4. Lebensjahr. Das Tragen der Orthese stellt eine erhebliche Belastung für den Säugling bzw. das Kleinkind dar, da sie dessen Beweglichkeit stark einschränkt.

Aus der US 4 157 876 ist eine weitere Orthese mit Gelenken bekannt. Die Gelenke sind mittels Schraubverbindungen in verschiedenen Befestigungslöchern einer stabartigen Verbindungsstruktur fixierbar.

Aus der US 3 477 426, US 4 040 416, DE 27 49 411 A1, US 5 364 463 sind Orthesen bekannt, bei denen die Verbindungsstrukturen zwischen Gelenken bzw. Befestigungseinrichtungen für die Füße insgesamt elastisch ausgeführt sind. Hierdurch werden besonders große Verformungen der Orthesen ermöglicht. Außerdem sind die Längen der insgesamt elastischen Verbindungsstrukturen nicht einstellbar.

Davon ausgehend liegt der Erfindung die Aufgabe zugrunde, eine Orthese zur Klumpfußbehandlung zur Verfügung zu stellen, die die Belastung des Patienten mindert und dennoch die Füße des Patienten in der gewünschten Ausrichtung fixiert.

Die Aufgabe wird durch eine Orthese mit den Merkmalen des Anspruches 1 gelöst. Vorteilhafte Ausgestaltungen der Orthese sind in den Unteransprüchen angegeben.

Die erfindungsgemäße Orthese zur Klumpfußbehandlung hat Befestigungseinrichtungen zum lösbaren Befestigen der Füße eines Patienten und eine die Befestigungseinrichtungen verbindende, diese in bestimmten Positionen in bezug aufeinander haltende Verbindungsstruktur und ist zumindest teilweise elastisch. Die Orthese ist **dadurch gekennzeichnet, daß** mindestens ein elastischer Abschnitt auf der von einer Befestigungseinrichtung abgewandten Seite mindestens eines Gelenkes zwischen diesem Gelenk und einem weiteren Abschnitt der Verbindungsstruktur angeordnet ist und/oder mindestens ein elastischer Abschnitt zwischen mindestens einem Gelenk und einer Befestigungseinrichtung angeordnet ist.

Die erfindungsgemäße Orthese ist ganz oder teilweise elastisch. Infolgedessen ist die Orthese elastisch verformbar. Der Kraftfluß zwischen den Füßen eines Patienten verläuft über die Befestigungseinrichtungen, an denen die Füße befestigt sind, und die Verbindungsstruktur. Bevorzugt ist die Verbindungsstruktur ganz oder teilweise elastisch. Zusätzlich oder statt dessen kann aber auch eine Befestigungseinrichtung oder können beide Befestigungseinrichtungen ganz oder teilweise elastisch sein. Somit kann die Orthese bei Belastung durch den Patienten etwas nachgeben, wobei die elastische Verformung der Orthese mit der Höhe der Belastung steigt. Hierdurch wird die Bewegungseinschränkung des Patienten beim Tragen der Orthese gemindert. Die elastische Verformbarkeit der Orthese ist so gewählt, daß die Orthese einem diese tragenden Säugling und/oder Kleinkind ermöglicht, die Füße bzw. Beine etwas zu bewegen (und zwar nicht nur parallel, sondern auch in Wechselbewegung) und nicht die Füße gleichsam starr fixiert. Aufgrund der Elastizität der Verbindungsstruktur und/oder mindestens einer der Befestigungseinrichtungen nimmt die Orthese bei Entlastung ihre Ausgangsform wieder an, so daß sichergestellt ist, daß die Füße in der therapeutisch gebotenen Ausrichtung gehalten werden. Die therapeutische Funktion der Orthese ist somit unvermindert. Infolge der verbesserten Trageeigenschaften ist zu erwarten, daß die Bereitschaft zur Anwendung der Orthese bei den Patienten und deren Eltern erhöht ist, wodurch ein erfolgreicher Abschluß der Therapie gefördert wird.

Die Befestigungseinrichtungen können verschieden ausgeführt sein. Gemäß einer Ausgestaltung weisen die Befestigungseinrichtungen Platten zum Abstützen der Sohlen eines Patienten auf. Es ist möglich, die Platten ganz oder teilweise elastisch auszuführen.

Mittels der Befestigungseinrichtungen können die Füße des Patienten ohne Schuhwerk direkt an der Orthese gehalten sein. Es ist aber auch möglich, mittels Befestigungseinrichtungen Schuhe des Patienten an der Orthese zu befestigen. Dabei können die Schuhe grundsätzlich beliebig ausgestaltet sein, z.B. als Halbschuhe oder als Sandalen. Gemäß einer Ausgestaltung weisen die Befestigungseinrichtungen Mittel zum lösbaren Befestigen von Schuhen des Patienten an den Platten auf. Gemäß einer weiteren Ausgestaltung weisen die Mittel zum lösbaren Befestigen Einrichtungen zum formschlüssigen Verbinden und/oder zum Verschnappen der Schuhe mit den Platten auf. Es ist möglich, die Mittel zum lösbaren Befestigen von Schuhen ganz oder teilweise elastisch auszuführen.

Grundsätzlich kann die Orthese mit einer Verbindungsstruktur ausgestattet sein, deren Geometrie zwar im Rahmen der elastischen Verformbarkeit veränderbar ist, die jedoch im übrigen nicht oder nur plastisch verformbar ist. Bei solchermaßen ausgestalteten Orthesen kann sichergestellt werden, daß die Befestigungseinrichtungen in den therapeutisch gewünschten Positionen angeordnet sind, indem z.B. die Orthese speziell nach den therapeutischen Erfordernissen gefertigt wird, die Verbindungsstruktur plastisch verformt wird oder die Orthese aus einem Vorrat von Orthesen mit verschiedenen Geometrien ausgewählt wird. Gemäß einer Ausgestaltung weist die Verbindungsstruktur mindestens ein Gelenk und eine Einrichtung zum lösbaren Fixieren des Gelenkes in verschiedenen Schwenkstellungen auf. Das Gelenk ermöglicht eine Positionierung der Befestigungseinrichtungen relativ zueinander entsprechend der therapeutisch gebotenen Anordnung der Füße des Patienten. Auch ist im Verlaufe einer Therapie ein Nachstellen des Gelenkes möglich.

Gemäß einer weiteren Ausgestaltung weist die Verbindungsstruktur zwei Gelenke, Einrichtungen zum lösbaren Fixieren der beiden Gelenke in verschiedenen Schwenkstellungen und einen Abschnitt der Verbindungsstruktur zwischen den beiden Gelenken auf. Durch die beiden Gelenke kann der Einstellbereich der Befestigungseinrichtungen relativ zueinander vergrößert werden. Gemäß einer weiteren Ausgestaltung weisen die beiden Gelenke jeweils ein Gelenkteil auf, das mit einer Befestigungseinrichtung verbunden ist. Bei dieser Ausgestaltung befinden sich die Gelenke in der Nähe der Füße, so daß deren Anordnung mittels der Gelenke genau einstellbar ist.

Die Verbindungsstruktur kann auf verschiedene Weise zumindest teilweise elastisch ausgerührt sein. Gemäß einer Ausgestaltung ist die Verbindungsstruktur insgesamt elastisch. Diese Ausgestaltung ist sowohl bei Orthesen möglich, die kein Gelenk aufweisen, als auch bei Orthesen, die mit mindestens einem Gelenk versehen sind. Die gesamte Verbindungsstruktur, die gegebenenfalls mindestens ein Gelenk umfaßt, ist dann elastisch ausgeführt.

Gemäß einer Ausgestaltung weist die Verbindungsstruktur mindestens einen elastischen Abschnitt auf. Dann ist die Verbindungsstruktur nur abschnittsweise elastisch. Im übrigen ist sie starr. Der elastische Abschnitt kann ein Abschnitt einer gelenkfreien Verbindungsstruktur oder einer Gelenke umfassenden Verbindungsstruktur sein. Der elastische Abschnitt kann insbesondere ein elastisches Gelenk (z.B. ein Schwenkgelenk mit elastischer Lagerschale) der Verbindungsstruktur sein.

Gemäß einer Ausgestaltung weist die Verbindungsstruktur außerhalb mindestens eines Gelenkes mindestens einen elastischen Abschnitt auf. Gemäß einer weiteren Ausgestaltung ist mindestens ein elastischer Abschnitt ein zwischen zwei Gelenken angeordneter Abschnitt der Verbindungsstruktur.

Gemäß einer Lösungsalternativen ist mindestens ein elastischer Abschnitt auf der von einer Befestigungseinrichtung abgewandten Seite mindestens eines Gelenkes zwischen diesem Gelenk und einem weiteren Abschnitt der Verbindungsstruktur angeordnet. Gemäß einer weiteren Ausgestaltung ist der weitere Abschnitt der Verbindungsstruktur starr. Gemäß einer weiteren Ausgestaltung ist zwischen zwei Gelenken ein starrer weiterer Abschnitt der Verbindungsstruktur angeordnet und zwischen jedem Gelenk und dem starren weiteren Abschnitt der Verbindungsstruktur ein elastischer Abschnitt angeordnet.

Gemäß einer weiteren Lösungsvariante, die auch gemeinsam mit der vorbeschriebenen Lösungsvariante eingesetzt werden kann, ist mindestens ein elastischer Abschnitt zwischen mindestens einem Gelenk und einer Befestigungseinrichtung angeordnet. Gemäß einer weiteren Ausgestaltung ist zwischen zwei Gelenken ein starrer Abschnitt der Verbindungsstruktur angeordnet und zwischen jedem Gelenk und einer Befestigungseinrichtung ein elastischer Abschnitt vorhanden.

Der elastische Abschnitt kann verschiedene Formen aufweisen, z.B. die Form eines U-, T-, C- oder Kastenprofiles. Gemäß einer Ausgestaltung ist der elastische Abschnitt zylindrisch. Gemäß einer weiteren Ausgestaltung ist er vollzylindrisch.

Der elastische Abschnitt kann verschieden mit der übrigen Verbindungsstruktur verbunden sein. Gemäß einer Ausgestaltung ist der elastische Abschnitt integral mit der Verbindungsstruktur ausgebildet. Gemäß einer anderen Ausgestaltung ist der elastische Abschnitt ein elastisches Bauteil, das mit mindestens einem weiteren Teil der Verbindungsstruktur zusammengebaut ist.

Verschiedene Ausführungen des elastischen Bauteiles sind möglich. Gemäß einer Ausgestaltung ist es eine Schraubenfeder und/oder ein elastomeres Bauteil. Insbesondere zum Schutz gegen Quetschungen ist die Schraubenfeder gemäß einer Ausgestaltung außen durch eine Hülle bedeckt. Als elastomeres Bauteil können z.B. Schwinggummis zum Einsatz kommen, die zur Lagerung bzw. Vibrationsdämpfung von Maschinen oder Motoren zum Einsatz kommen.

Die Verbindung des elastischen Bauteiles mit den übrigen Teilen der Verbindungsstruktur bzw. den Befestigungseinrichtungen kann auf verschiedene Weise erfolgen. Die Schraubenfeder ist beispielsweise mit beiden Enden fest mit verschiedenen Abschnitten der Verbindungsstruktur und/oder einer Befestigungseinrichtung verbunden. Gemäß einer Ausgestaltung weist das elastische Bauteil Gewindebolzen und/oder Gewindebuchsen zum Verbinden mit benachbarten Abschnitten der Verbindungsstruktur und/oder der Befestigungseinrichtung auf. Gemäß einer weiteren Ausgestaltung sind die Gewindebolzen und/oder Gewindebuchsen in dem elastomeren Bauteil verankert.

Der elastische Abschnitt ist z.B. aus Naturkautschuk oder aus synthetischem Kautschuk oder aus thermoplastischem Elastomer hergestellt. Es ist beispielsweise aus Gummi oder Silikonkautschuk. Die Gewindebolzen und/oder Gewindehülsen des elastischen Bauteils sind z.B. in ein Bauteil aus Gummi oder Silikonkautschuk einvulkanisiert. Bei den Gewindebolzen und/oder Gewindebuchsen handelt es sich z.B. um Metall- und/oder Kunststoffbauteile. Der elastische Abschnitt aus thermoplastischem Elastomer ist z.B. integral mit einer Verbindungsstruktur aus einem geeigneten Termoplasten gespritzt oder über Gewindebolzen und/oder Gewindebuchsen mit der Verbindungsstruktur verbunden.

Gemäß einer Ausgestaltung ist dem elastischen Abschnitt eine Einrichtung zum Begrenzen der Verformbarkeit des elastischen Abschnittes zugeordnet. Die Einrichtung zum Begrenzen der Verformbarkeit ist z.B. eine Einrichtung, die eine Längung des elastischen Abschnittes in mindestens einer Richtung begrenzt oder verhindert und/oder eine Einrichtung, die ein Verbiegen des elastischen Abschnittes in mindestens einer Ebene einschränkt oder verhindert und/oder eine Einrichtung, die eine Torsion des elastischen Abschnittes um mindestens eine Achse einschränkt oder verhindert. Die Einrichtung zum Begrenzen der Verformbarkeit verhindert therapeutisch unerwünschte Verformungen des elastischen Abschnittes unter Belastung durch den Patienten.

Gemäß einer Ausgestaltung weist die Einrichtung zum Begrenzen der Verformbarkeit eine den elastischen Abschnitt zumindest teilweise umgebende Einfassung auf. Die Einfassung begrenzt die Verformbarkeit der von ihr umgebenen Bereiche des elastischen Abschnittes. Hierzu ist sie z.B. starr oder starrer als der elastische Abschnitt. Gemäß einer weiteren Ausgestaltung deckt die Einfassung den elastischen Abschnitt an verschiedenen Umfangspositionen in unterschiedlichem Ausmaß ab. Hierdurch kann die Verformbarkeit des elastischen Abschnittes in verschiedenen Richtungen unterschiedlich begrenzt werden, entsprechend den therapeutischen Anforderungen. Gemäß einer weiteren Ausgestaltung weist die Einfassung einen Tiefpunkt und auf der diametral gegenüberliegenden Seite einen Hochpunkt und dazwischen einen vom Tiefpunkt zum Hochpunkt allmählich ansteigenden Verlauf auf. Gemäß einer anderen Ausgestaltung ist die Einfassung zylindrisch.

Bei einer mindestens ein Gelenk aufweisenden Ausführung der Orthese weist gemäß einer weiteren Ausgestaltung mindestens ein Gelenk ein Gelenkelement mit einer Einfassung auf, die einen elastischen Abschnitt der Verbindungsstruktur zumindest teilweise aufnimmt.

Das Gelenk kann verschieden ausgeführt sein. Beispielsweise ist es ein einfaches Schwenkgelenk. Gemäß einem anderen Beispiel ist das Schwenkgelenk ein doppeltes Schwenkgelenk mit zwei zueinander senkrechten Schwenkebene. Das Schwenkgelenk ermöglicht ein Schwenken im Bereich der Dorsalextension und/oder der Abduktion.

Gemäß einer Ausgestaltung weist mindestens ein Gelenk eine Kugel oder einen Kugelabschnitt auf, auf der oder dem eine Kugelschale schwenkbar angeordnet ist. Bei der Ausführung eines Kugelgelenkes mit einem Kugelabschnitt ist die Größe gegenüber einer Ausführung mit einer Kugel reduziert. Bei einer Orthese mit zwei Gelenken sind im Hinblick auf den therapeutisch geforderten Einstellwinkel von maximal 20° in Bereich der Dorsalextension Kugelabschnitte ausreichend. Diese sind z.B. Halbkugeln. Gemäß einer weiteren Ausgestaltung weisen die Kugel oder der Kugelabschnitt und/oder die Kugelschale meridional erstreckte Nuten auf, in die eine von der Kugelschale und/oder der Kugel und/oder dem Kugelabschnitt vorstehende Rippe eingreift. Nut und Rippe lassen lediglich eine Kippbewegung zu, zwecks Einstellung der Dorsalextension. Die Abduktion ist durch Schwenken der Kugel oder des Kugelabschnittes bezüglich des angrenzenden Abschnittes der Verbindungsstruktur einstellbar.

Gemäß einer Ausgestaltung weist die Einrichtung zum lösbaren Fixieren des Gelenkes eine Klemmverbindung auf, mittels der die Gelenkteile in verschiedenen Schwenkpositionen bezüglich einander festklemmbar sind. Gemäß einer weiteren Ausgestaltung weist die Einrichtung zum lösbaren Fixieren des Gelenkes in verschiedenen Schwenkstellungen eine Schraubverbindung auf, welche eine Durchgangslöcher der Kugel oder des Kugelabschnittes oder der Kugelschale durchgreifende Schraube hat, wobei mindestens eines der Durchgangslöcher eine die Schraube mit Abstand umgebende Begrenzung aufweist. Das die Schraube mit Abstand umgebende Durchgangsloch ermöglicht ein begrenztes Schwenken der Kugelschale bezüglich des Kugelabschnittes oder der Kugel.

Gemäß einer weiteren Ausgestaltung ist die Kugel oder der Kugelabschnitt oder die Kugelschale drehbar und in bestimmten Drehstellungen lösbar fixierbar mit dem Verbindungsträger zwischen den Gelenken verbunden.

Zur Vermeidung therapeutisch nicht zulässiger Einstellungen weist gemäß einer Ausgestaltung mindestens ein Gelenk eine Einrichtung zum Begrenzen des Schwenkwinkels in mindestens einer Schwenkebene auf.

Gemäß einer Ausgestaltung weist die Verbindungsstruktur zwei parallele Stangen und mindestens eine Klemmvorrichtung und/oder Schraubvorrichtung zum lösbaren Fixieren der parallelen Stangen in verschiedenen Stellungen zueinander auf. Dies ermöglicht ein Einstellen des Abstandes der Befestigungseinrichtungen voneinander, in Anpassung an den jeweiligen Patienten.

Gemäß einer Ausgestaltung ist die Verbindungsstruktur in mindestens einer Vorzugsebene elastisch und in mindestens einer weiteren Ebene nicht oder weniger elastisch. Die Elastizität in einer Vorzugsebene kann z.B. durch Integration einer fahrradkettenähnlichen Struktur in die Verbindungsstruktur erzeugt werden, welche eine Verformung bevorzugt in einer Ebene ermöglicht. Entsprechendes gilt für die Ausgestaltung mindestens eines elastischen Abschnittes der Verbindungsstruktur.

Nachfolgend wird die Erfindung anhand der anliegenden Zeichnungen von Ausführungsbeispielen näher erläutert. In den Zeichnungen zeigen:
- Fig. 1: eine Orthese mit zwei Gelenken und elastomeren Bau- teilen zwischen den Gelenken und Platten in einer Per- spektivansicht schräg von oben und von vom;
- Fig. 2: dieselbe Orthese in einer Perspektivansicht schräg von unten und von vom;
- Fig. 3: einen durch die Verbindungsstruktur abgetrennten und teilweise geschnittenen Teil derselben Orthese in einer Perspektivansicht schräg von oben und von hinten;
- Fig. 4: einen durch die Verbindungsstruktur abgeschnittene Teil derselben Orthese ohne das elastomere Bauteil in einer Ansicht aus derselben Perspektive;
- Fig. 5: dieselbe Orthese mit einer anderen Längeneinstellung der Verbindungsstruktur in einer Perspektivansicht schräg von oben;
- Fig. 6: eine weitere Orthese mit zwei Gelenken an den Enden einer Verbindungsstruktur und elastomeren Bauteilen zwischen den Gelenken und einem zentralen Abschnitt der Verbindungsstruktur in einer Perspektivansicht schräg von oben;
- Fig. 7: dieselbe Orthese in einer Perspektivansicht schräg von der Seite;
- Fig. 8: elastomeres Bauteil mit den angrenzenden Abschnitten der Verbindungsstruktur in einer vergrößerten Perspek- tivansicht schräg unten;
- Fig. 9: eine weitere Orthese mit elastischen Abschnitten der Ver- bindungsstruktur in einer grobschematischen Vorder- ansicht;
- Fig. 10: eine weitere Orthese mit einer insgesamt elastischen Ver- bindungsstruktur in einer grobschematischen Vorder- ansicht;
- Fig. 11: eine weitere Orthese mit zwei Gelenken und elastomeren Bauteilen zwischen den Gelenken und einem starren Ab- schnitt der Verbindungsstruktur in einer Perspektiv- ansicht schräg von oben und von vom;
- Fig. 12: ein elastomeres Bauteil der Orthese von Fig. 11 in einer Perspektivansicht schräg von oben und von vom;
- Fig. 13: eine weitere Orthese mit zwei Gelenken und elastomeren Bauteilen zwischen den Gelenken und einem starren Ab- schnitt der Verbindungsstruktur in einer Perspektiv- ansicht schräg von unten und von hinten;
- Fig. 14: Gelenk mit Platte und Halteeinrichtung für ein elasto- meres Bauteil derselben Orthese in einer vergrößerten Detailansicht;
- Fig. 15: ein elastomeres Bauteil derselben Orthese in einer Perspektivansicht schräg von oben und von der Seite.

Bei der nachfolgenden Beschreibung verschiedener Ausführungsbeispiele sind übereinstimmende Elemente mit denselben Bezugsziffern versehen.

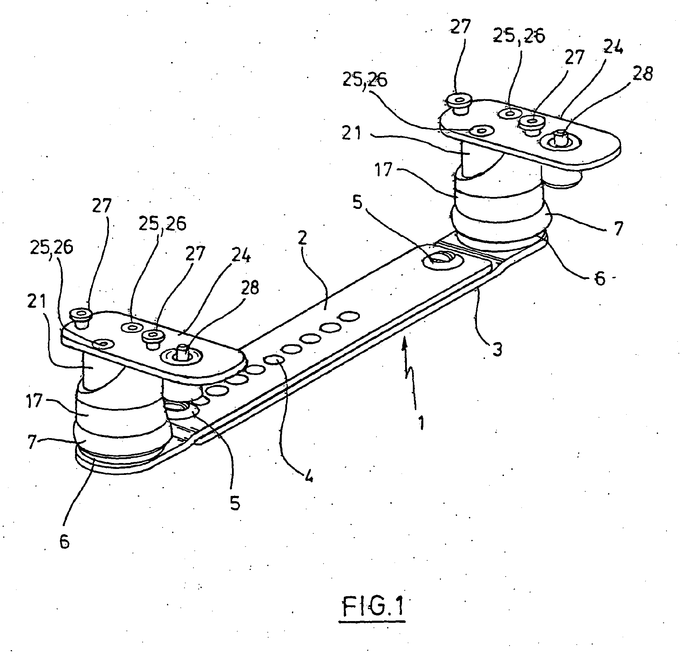

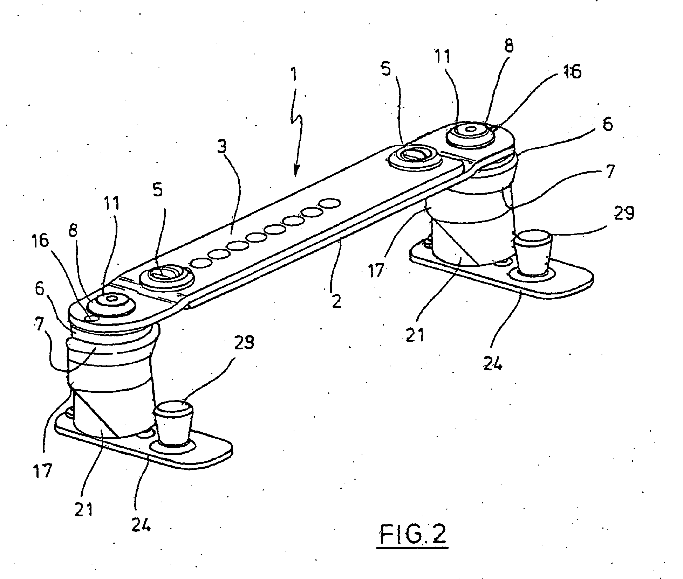

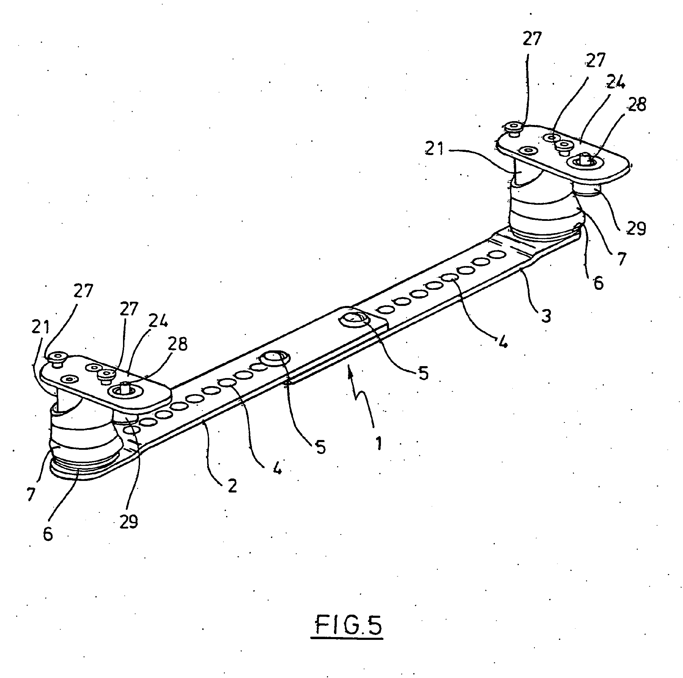

Die Orthese von Fig. 1 bis 5 weist eine Verbindungsstruktur 1 auf, die zwei parallele Stangen 2, 3, z.B. aus starrem Kunststoff oder Stahl, hat. Jede Stange 2, 3 weist eine Reihe von Löchern 4 auf, die dazu dienen, die beiden Stangen im Überlappungsbereich durch eingeführte Schraubverbindungen 5 miteinander zu verbinden. Durch unterschiedliche Überlappung der Stangen 2, 3 und Setzen der Schraubverbindungen 5 in verschiedene Löcher 4, ist die Länge der Verbindungsstruktur einstellbar (vgl. Fig. 1 und 5).

An den Enden der Stangen 2, 3 sind Kugelabschnitte 6 angeordnet. Auf den Kugelabschnitten 6 sitzen Kugelschalen 7.

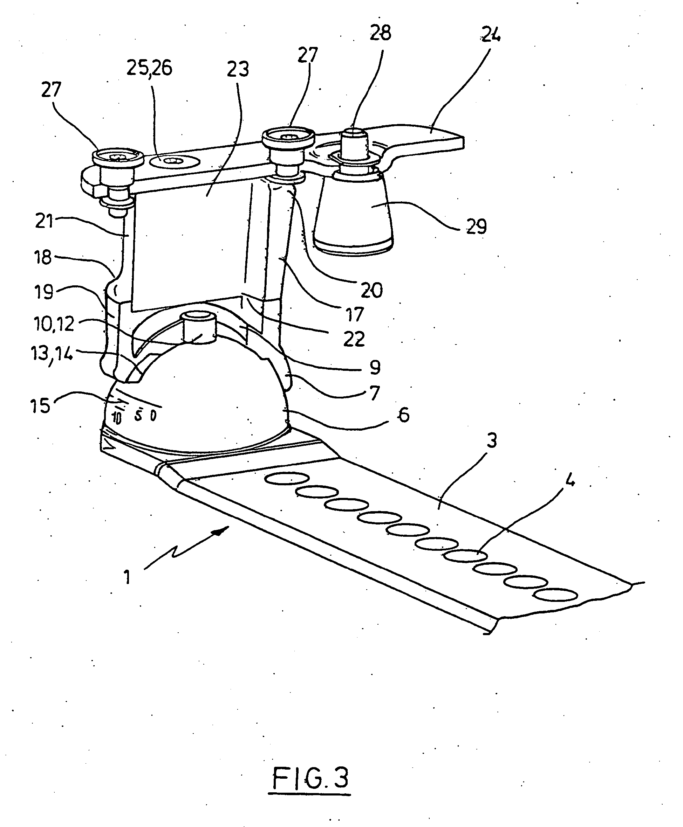

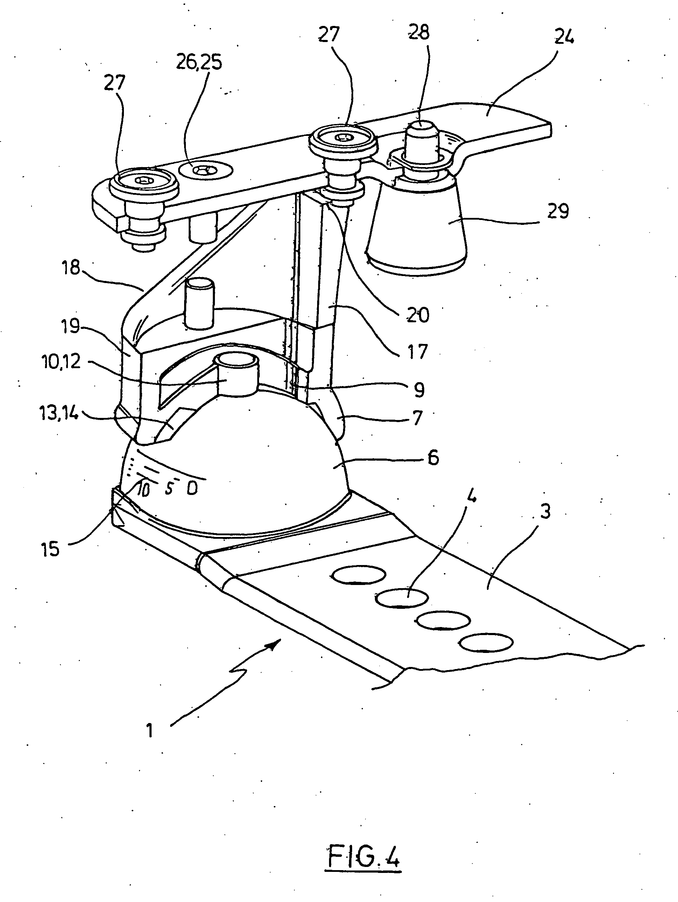

Die Kugelabschnitte 6 weisen ein nicht gezeigte radiale Durchgangslöcher auf, die mit Durchgangslöchern 8 an den Enden der Stangen 2, 3 fluchten. Ferner haben die Kugelschalen 7 jeweils einen meridional verlaufenden Schlitz 9 (vgl. Fig. 3, 4).

Weitere Schraubenverbindungen sind mit jeweils einer Schraube 10 durch die Durchgangslöcher der Kugelabschnitte 6 hindurch und in die Schlitze 9 hineingeführt. Die Schraubenköpfe 11 sitzen an der Unterseite der Stangen 2, 3 und Schraubenmuttern 12 sind in den Schlitzen 9 angeordnet.

Von den Innenseiten der Kugelschalen 7 stehen meridional verlaufende Rippen 13 vor, die in meridionale Nuten 14 der Kugelabschnitte 6 eingreifen. Infolgedessen sind die Kugelschalen 7 nur in Meridionalrichtung bezüglich der Kugelabschnitte 6 schwenkbar.

Wenn die Schraubenverbindungen 10 angezogen sind, sind die Kugelschalen 7 in bestimmten Schwenkstellungen an den Kugelabschnitten 6 gesichert. Außerdem sind die Kugelabschnitte 6 in bestimmten Drehstellungen an den Stangen 2, 3 gesichert. Nach Lockern der Schraubenverbindung 10 ist es möglich, die Kugelabschnitte 6 auf den Stangen 2, 3 zu drehen und die Kugelschalen 7 bezüglich der Kugelabschnitte 6 zu schwenken.

Auf der Außenseite der Kugelabschnitte 6 sind vorn Gradeinteilungen 15 angebracht, die es ermöglichen, den Schwenkwinkel der Kugelschalen 7 bezüglich der Kugelabschnitte 6 abzulesen.

Die Kugelabschnitte 6 haben an der Unterseite eine nicht gezeigte Winkeleinteilung, die durch Fenster 16 in den Unterseiten der Stangen 2, 3 ablesbar ist. Dies ermöglicht ein Ablesen der Drehwinkel des Kugelabschnitte 6 bezüglich der Stangen 2, 3.

Die Kugelschalen tragen oben zylindrische Einfassungen 17. Diese weisen jeweils einen oberen Rand 18 auf, der diametral gegenüberliegend in der Schwenkebene der Kugelschale 7 bezüglich des Kugelabschnittes 6 einen Tiefpunkt 19 und einen Hochpunkt 20 aufweist. Vom Tiefpunkt 19 zum Hochpunkt 20 hin steigt der obere Rand 18 auf beiden Seiten der Schwenkebene allmählich an.

In jede Einfassung 17 ist ein vollzylindrisches elastomeres Bauteil 21 eingesetzt. Dieses ist an der Unterseite 22 auf nicht gezeigte Weise fest mit der Kugelschale 7 verbunden, beispielsweise über einen in die Unterseite 22 integrierten Schraubenbolzen oder durch Einkleben.

An der gegenüberliegenden Seite 23 ist jedes elastomere Bauteil 21 ebenfalls fest mit einer Platte 24 z.B. aus starrem Kunststoff oder Stahl verbunden. Jede Platte 24 hat zwei Durchgangslöcher 25, durch die zwei Schrauben 26 hindurchgeführt sind, die in nicht gezeigte Gewindebuchsen eingreifen, die in die Seiten 23 integriert sind.

Die elastomeren Bauteile 21 sind z.B. aus einem Naturkautschuk oder aus einem Synthesekautschuk hergestellt. Es ist beispielsweise aus Gummi.

Ferner sind die Platten 24 jeweils mit zwei Nutensteinen 27 versehen die nach oben vorstehen.

Schließlich weisen die Platten 24 jeweils einen verfederten Raststift 28 auf, der nach oben von der Platte 24 vorsteht. Der Raststift 28 hat einen nach unten vorstehenden Betätigungsknopf 29. Durch die nicht gezeigte Feder ist er so verfedert, daß der Raststift 28 die Tendenz hat, oben über die Platte 24 hinauszustehen.

Bei der Anwendung der Orthese wird der Abstand zwischen den beiden Gelenken 6, 7 an den beiden Enden der Verbindungsstruktur 1 eingestellt.

Nach Lösen der Schraubenverbindungen 10 werden außerdem die therapeutisch geforderten Kippwinkel und Drehwinkel der Gelenke 6, 7 eingestellt und durch Anziehen der Schraubenverbindungen 10 fixiert.

Ferner werden Schuhe des Patienten mit den Platten 24 verbunden. Hierfür weisen die Schuhe Nuten auf, in denen die Nutensteine 27 zum Eingriff kommen und Rastlöcher, in die die verfederten Stifte 28 einrasten, um die Nutensteine 27 an einem Herausrutschen aus den Nuten zu hindern.

Der Patient ist schließlich über beide Schuhe mit der Orthese verbunden.

Die elastomeren Bauteile 21 ermöglichen eine gewisse eingeschränkte Beweglichkeit nach vom und zu den Seiten hin, wobei die Beweglichkeit durch den Verlauf der oberen Ränder 18 der Einfassungen 17 begrenzt ist.

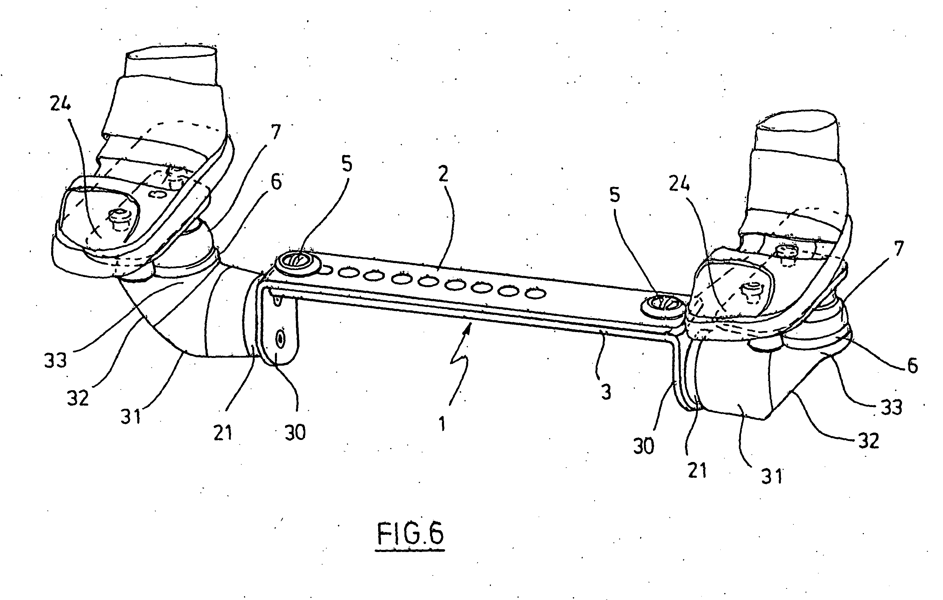

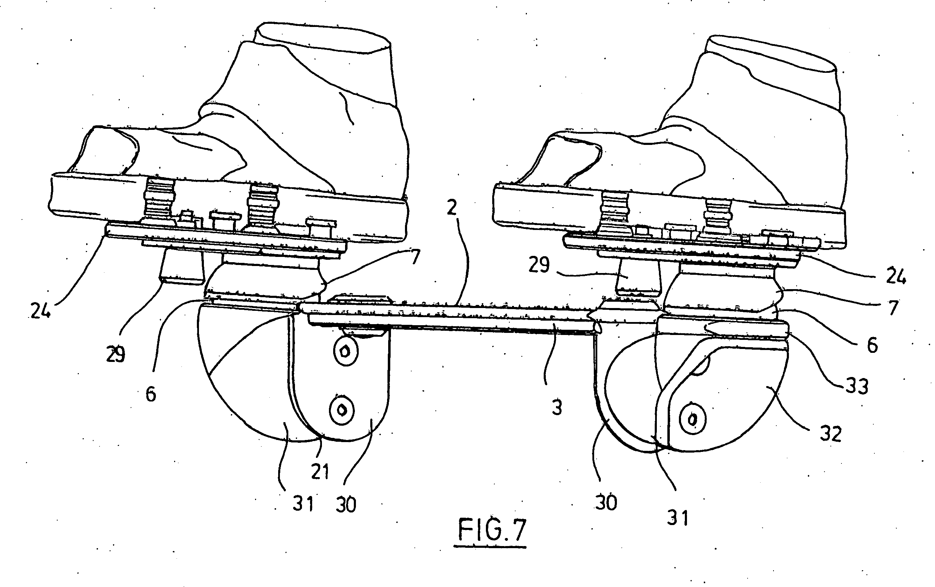

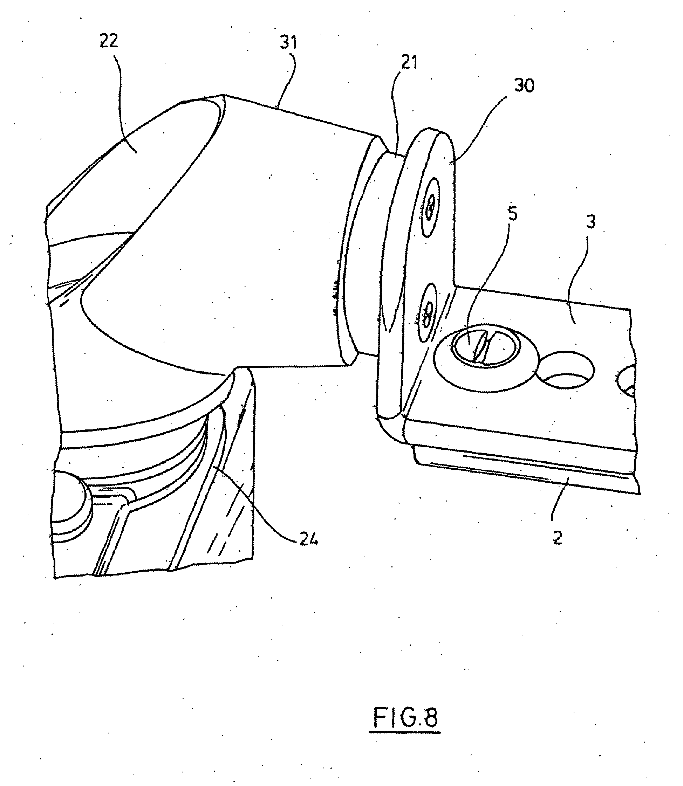

Die Orthese gemäß Fig. 6 bis 8 unterscheidet sich von der vorbeschriebenen dadurch, daß die Verbindungsstruktur 1 an den Enden Abwinklungen 30 aufweisen, an denen jeweils ein elastomeres Bauteil 21 mit Zylinderform befestigt ist.

Jedes elastomere Bauteil 21 trägt eine zylindrische Einfassung 31, die' jeweils endseitig mit einer Konsole 32 verbunden sind. Die Konsolen 32 haben oben jeweils einen Lagerteller 33, auf dem jeweils ein Gelenk 6, 7 positioniert ist, das oben eine Platte 24 trägt.

Durch die Anordnung der elastischen Bauteile 21 an den Enden der Verbindungsstruktur 1 werden die Aufstandsflächen der Fußsohlen abgesenkt, so daß diese näher an den Fußboden herankommen, so daß das Tragen der Orthese weniger belastend ist. Des weiteren wird durch die Anordnungen der elastischen Bauteile 21 die Gefahr des Umknickens des Patienten vermindert.

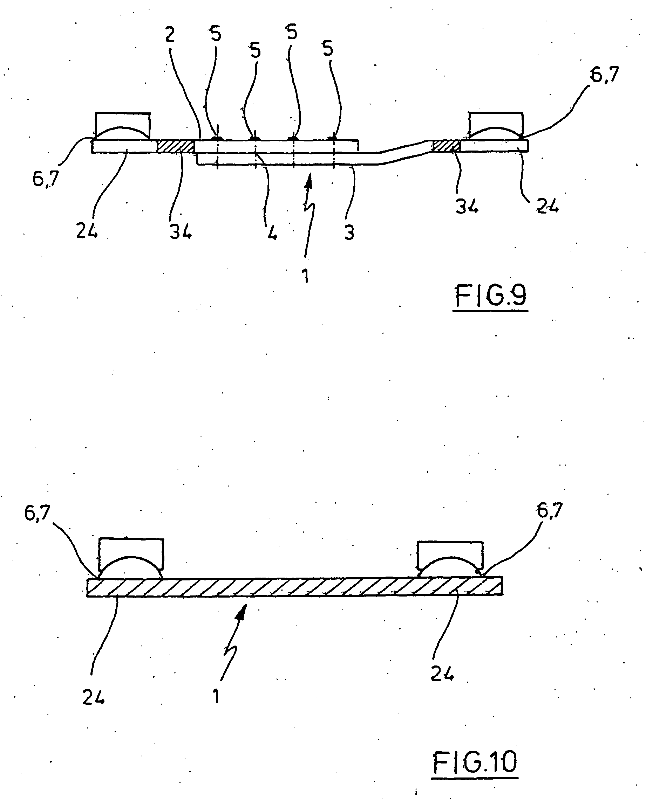

Bei der Ausgestaltung gemäß Fig. 9 ist lediglich eine Längeneinstellbarkeit der Verbindungsstruktur 1 gegeben, indem die Schraubverbindungen 5 in verschiedenen Überlappungsstellungen der Stangen in die Durchgangslöcher 4 gesetzt werden. Jede Stange 2, 3 der Verbindungsstruktur 1 ist grundsätzlich starr, hat jedoch einen elastischen Abschnitt 34, der integral mit der Stange 2, 3 ausgebildet ist. Der elastische Abschnitt 34 ist beispielsweise aus einem thermoplastischen Elastomer und die Stange 2, 3 bzw. die gesamte Verbindungsstruktur 1 im übrigen aus einem Thermoplasten. Alternativ kann der elastische Abschnitt mit der Stange verschraubt sein und aus -einem Elastomer bestehen.

Die Enden der Stangen 2, 3 können zur Befestigung von Gelenken 6, 7 aus einem starren Thermoplast oder metallisch ausgerührt sein oder alternativ kann der elastische Abschnitt aus Befestigungsgrundlage für Gelenke 6, 7 dienen.

Gemäß Fig. 10 ist die gesamte stangenförmige Verbindungsstruktur 1 flexibel, z.B. durch Ausbildung derselben aus Gummi oder einem thermoplastischen Elastomer. Auch ist es möglich, die Verbindungsstruktur 1 aus mehreren Lagen mit unterschiedlichem Härtegrad auszuführen bzw. mit unterschiedlicher Flexibilität, so daß sich eine insgesamt flexible Verbindungsstruktur 1 ergibt. Auch bei der Ausführung von Fig. 10 können die Enden der Verbindungsstruktur 1 Gelenke 6, 7 aufweisen.

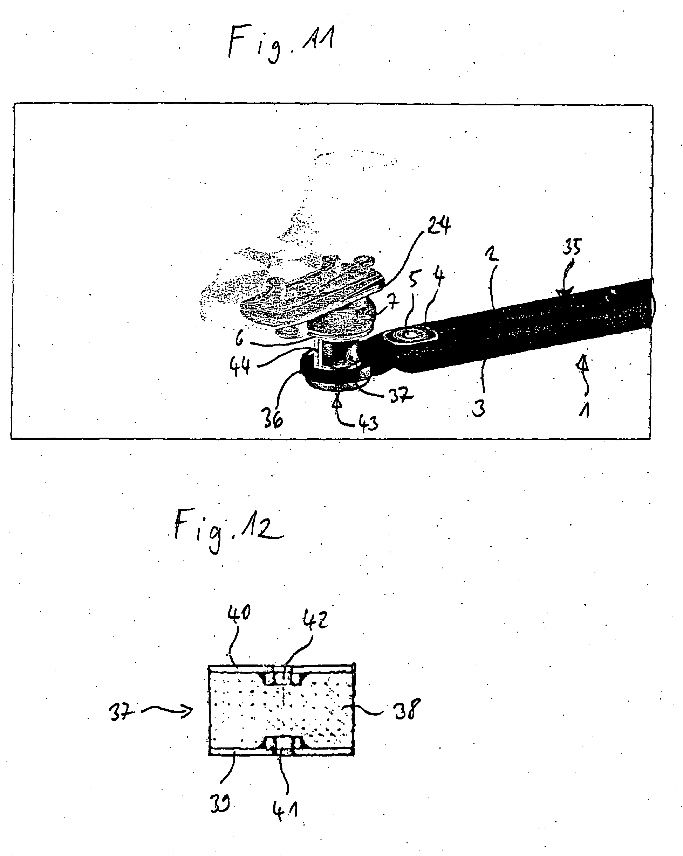

Bei der Ausführung von Fig. 11 und 12 weist die Verbindungsstruktur 1 zwei Stangen 2, 3 auf, die an den einander zugewandten Seiten profiliert und aneinander geführt sind. Jede Stange 2, 3 hat ein Loch 4 und ein Langloch 35. Jeweils eine Schraubverbindung 5 sitzt in einem Loch 4 der einen Stange 2, 3 und in einem Langloch 35 der anderen Stange 3, 2. Bei gelockerten Schraubverbindungen 5 ist die Länge der Verbindungsstruktur 1 einstellbar und die eingestellte Länge ist dann durch Anziehen der Schraubverbindungen fixierbar.

An den Enden sind die Stangen 2, 3 mit ringscheibenförmigen Halteelemente 36 versehen, in denen elastomere Bauteile 37 angeordnet sind. Diese haben gemäß Fig. 12 ein zylindrisches Element 38 aus einem elastomeren Material, das an den beiden Stirnseiten befestigte, kreisringförmige Scheiben 39, 40 mit Gewindebohrungen 41, 42 aufweist. Die Scheibe 39 liegt auf der Oberseite des Halteelementes 36 auf. Eine Schraube 43 ist von unten durch die Öffnung des Halteelementes 36 hindurchgesteckt und in die Gewindebohrung 41 eingeschraubt.

In die Gewindebohrung 42 ist jeweils eine durch eine Bohrung des Kugelabschnittes 6 gesteckte und mit dem Schraubenkopf 11 in einem Schlitz 9 angeordnete Schraube 10 eingeschraubt. Auf jedem Kugelabschnitt 6 ist eine Kugelschale 7 schwenkbar fixiert. Der Aufbau de jeweils einen Kugelabschnitt 6 und eine Kugelschale 7 umfassenden Gelenke 6, 7 entspricht grundsätzlich dem Aufbau der Gelenke 6, 7 des Ausführungsbeispieles von Fig. 1 bis 5. Bei dem vorliegenden Ausführungsbeispiel ist jedoch die Kugelschale 7 oben nicht mit einer zylindrischen Einfassung 17 versehen, sondern direkt mit einer Platte 24 verbunden.

Ferner ist in jeden Kugelabschnitt 6 ein Stift 44 eingeschraubt, der vom unteren Rand des Kugelabschnittes 6 vorsteht und in der Schwenkebene der Kugelschale 7 bezüglich des Kugelabschnittes 6 angeordnet ist. Hierbei handelt es sich um die Meridionalebene, die durch die Rippen 13 und Nuten 14 definiert ist (vgl. Fig. 3, 4). Der Stift 44 stützt sich am oberen Rand des Halteelements 36 ab.

Die Gelenke 6, 7 sind wie bei dem Ausführungsbeispiel von Fig. 1 bis 5 einstellbar. Der Stift 44 begrenzt die Schwenkbewegung der Platten 24 bzw. der Füße des Patienten 6, 7 nach vom. Zu den Seiten und nach hinten sind die Gelenke 6, 7 mit den daran befestigten Platten 24 schwenkbar. Die elastomeren Bauteile 37 stellen die Platten 24 bei Entlastung in die Ausgangslage zurück.

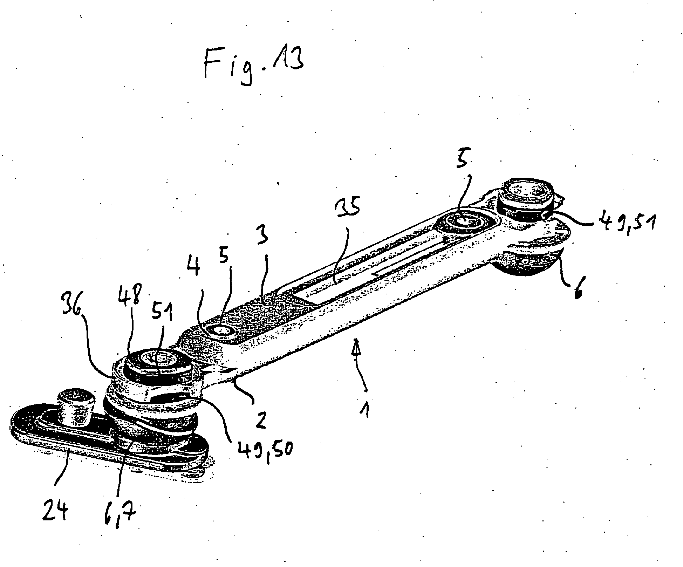

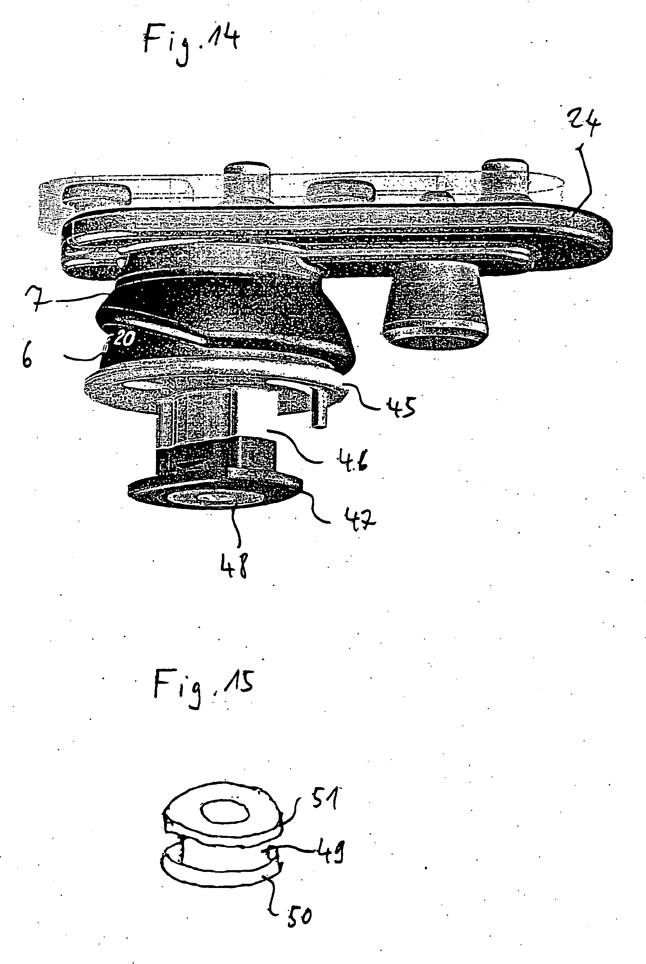

Bei der Ausführung von Fig. 13 bis 15 ist an der Unterseite jedes Kugelabschnittes 6 der beiden Gelenke 6, 7 ein Halter 45 für eine Hülse aus einem elastomeren Material angeordnet. Hierfür hat der Halter 45 einen zentralen Zapfen 46, auf den die Hülse aufsetzbar ist. Der Zapfen 46 trägt am Ende eine Fixierplatte 47, die mittels einer Schraube 48 an dem Zapfen 46 fixiert ist.

Die Halter 45 sind mit dem Zapfen 46 in ovaläre Öffnungen der Halteelemente 36 der Stange 1 eingesetzt. Zwischen den Halteelementen 36 und den Haltern 45 sind am Außenumfang ovaläre Hülsen 49 aus elastomerem Material angeordnet und mittels der Fixierplatte 47 gesichert. Die Hülsen 49 haben an der Oberseite und der Unterseite der Halteelemente 36 überstehende Ränder 50, 51 damit sie nicht herausrutschen. Bei der Montage werden zunächst die Hülsen 49 auf die Zapfen 46 gesetzt und dann von oben in die Öffnungen der Halteelemente eingepresst. Beim Einsetzen werden die Ränder 51 der Hülsen 49 radial zusammengepreßt und danach weiten sie sich wieder radial auf. Schließlich werden die Ränder 51 durch die Fixierplatten 47 abgestützt, so daß sie nicht aus den Öffnungen herausgezogen werden können.

Die Platten 24 sind in verschiedenen Richtungen bezüglich der Verbindungsstruktur 1 schwenkbar, wobei die elastomeren Bauteile 49 die Platten 24 in die Ausgangsstellung zurückzustellen.

## Patentansprüche

1. Orthese zur Klumpfußbehandlung, die Befestigungseinrichtungen (24, 27, 28) zum Befestigen der Füße eines Patienten und eine die Befestigungseinrichtungen (24, 27, 28) verbindende, diese in bestimmten Positionen in bezug aufeinander haltenden Verbindungsstruktur (1) aufweist und die zumindest teilweise elastisch ist, **dadurch gekennzeichnet, daß** mindestens ein elastischer Abschnitt (37) auf der von einer Befestigungseinrichtung (24) abgewandten Seite mindestens eines Gelenkes (6, 7) zwischen diesem Gelenk (6, 7) und einem weiteren Abschnitt der Verbindungsstruktur (1) angeordnet ist und/oder daß mindestens ein elastischer Abschnitt (21, 34) zwischen mindestens einem Gelenk (6, 7 und einer Befestigungseinrichtung (24, 27, 28) angeordnet ist.

2. Orthese nach Anspruch 1, die Befestigungseinrichtungen (27, 28) zum lösbaren Befestigen der Schuhe des Patienten aufweist.

3. Orthese nach Anspruch 2, bei der die Befestigungseinrichtungen Mittel zum lösbaren Befestigen (27, 28) von Schuhen des Patienten an Platten (24) aufweisen.

4. Orthese nach Anspruch 3, bei der die Mittel zum lösbaren Befestigen (27, 28) Einrichtungen zum formschlüssigen Verbinden und/oder zum Verschnappen (27, 28) der Schuhe mit den Platten (24) aufweisen.

5. Orthese nach einem der Ansprüche 1 bis 4, bei der die Verbindungsstruktur (1) mindestens ein Gelenk (6, 7) und eine Einrichtung zum lösbaren Fixieren (10) des Gelenkes in verschiedenen Schwenkstellungen aufweist

6. Orthese nach Anspruch 5, bei der die Verbindungsstruktur zwei Gelenke (6, 7), Einrichtungen zum lösbaren Fixieren (10) der beiden Gelenke in verschiedenen Schwenkstellungen und einen Abschnitt (2, 3) der Verbindungsstruktur (1) zwischen den beiden Gelenken aufweist.

7. Orthese nach Anspruch 6, bei der die beiden Gelenke (6, 7) jeweils ein Gelenkteil (7) aufweisen, das mit einer Befestigungseinrichtung (24, 27, 28) verbunden ist.

8. Orthese nach einem der Ansprüche 1 bis 7, bei der die Verbindungsstruktur (1) insgesamt elastisch ist.

9. Orthese nach einem der Ansprüche 1 bis 7, bei der die Verbindungsstruktur (1) mindestens einen elastischen Abschnitt (21, 34) aufweist.

10. Orthese nach einem der Ansprüche 5 bis 9, bei der die Verbindungsstruktur (1) außerhalb mindestens eines Gelenkes (6, 7) mindestens einen elastischen Abschnitt (21, 34) aufweist.

11. Orthese nach Anspruch 9 oder 10, bei der mindestens ein elastischer Abschnitt (21, 34) ein zwischen zwei Gelenken (6, 7) angeordneter Abschnitt der Verbindungsstruktur (1) ist

12. Orthese nach einem der Ansprüche 1 bis 11, bei der der weitere Abschnitt (2, 3) der Verbindungsstruktur (1) starr ist.

13. Orthese nach einem der Ansprüche 1 bis 12, bei der zwischen zwei Gelenken (6, 7) ein starrer weiterer Abschnitt (2, 3) der Verbindungsstruktur (1) angeordnet ist und zwischen jedem Gelenk (6, 7) und dem starren weiteren Abschnitt (2, 3) der Verbindungsstruktur (1) ein elastischer Abschnitt (36) angeordnet ist.

14. Orthesen nach einem der Ansprüche 1 bis 13, bei der zwischen zwei Gelenken (6, 7) ein starrer Abschnitt der Verbindungsstruktur (1) angeordnet ist und zwischen jedem Gelenk (6, 7) und einer Befestigungseinrichtung (24) ein elastischer Abschnitt vorhanden ist.

15. Orthese nach einem der Ansprüche 9 bis 14, bei der der elastische Abschnitt (21) zylindrisch ist.

16. Orthese nach einem der Ansprüche 1 bis 15, bei der der elastische Abschnitt (34) integral mit der Verbindungsstruktur ausgebildet ist.

17. Orthese nach einem der Ansprüche 1 bis 16, bei der der elastische Abschnitt ein elastisches Bauteil (21) ist.

18. Orthese nach Anspruch 17, bei der das elastische Bauteil (21) eine Schraubenfeder und/oder ein elastomeres Bauteil umfaßt.

19. Orthese nach Anspruch 17 oder 18, bei der das elastische Bauteil (21) Gewindebolzen und/oder Gewindebuchsen zum Verbinden mit benachbarten Abschnitten der Verbindungsstruktur und/oder der Befestigungseinrichtung aufweist.

20. Orthese nach Anspruch 19, bei der die Gewindebolzen und/oder Gewindebuchsen in dem elastomeren Bauteil (21) verankert sind,

21. Orthese nach einem der Ansprüche 9 bis 20, bei der dem elastischen Abschnitt (21) eine Einrichtung zum Begrenzen (17) der Verformbarkeit des elastischen Abschnittes zugeordnet ist.

22. Orthese nach Anspruch 21, bei der die Einrichtung zum Begrenzen (17) der Verformbarkeit eine den elastischen Abschnitt (21) zumindest teilweise umgebende Einfassung aufweiset.

23. Orthese nach Anspruch 22, bei der die Einfassung (17) den elastischen Abschnitt (21) an verschiedenen Umfangspositionen in unterschiedlichem Ausmaß abdeckt.

24. Orthese nach einem der Anspruche 22 oder 23, bei der die Einfassung (17) einen Tiefpunkt und auf der diametral gegenüberliegenden Seite einen Hochpunkt und dazwischen einen vom Tiefpunkt zum Hochpunkt allmählich ansteigenden Verlauf aufweist.

25. Orthese nach einem der Ansprüche 22 bis 24, bei der die Einfassung (17) zylindrisch ist.

26. Orthese nach Anspruch 22 bis 25, bei der mindestens ein Gelenk (6, 7) ein Celenkelement (7) mit einer Einfassung (17) aufweist, die einen elastischen Abschnitt (21) zumindest teilweise aufnimmt.

27. Orthese nach einem der Ansprüche 6 bis 26, bei der mindestens ein Gelenk (6, 7) ein Schwenkgelenk ist.

28. Orthese nach Anspruch 27, bei der das Schwenkgelenk (6, 7) zwei zueinander senkrechte Schwenkebenen aufweist.

29. Orthese nach einem der Ansprüche 6 bis 28, bei der mindestens ein Gelenk (6, 7) eine Kugel oder einen Kugelabschnitt (6) aufweiset, auf der oder dem eine Kugelschale (7) schwenkbar angeordnet ist.

30. Orthese nach Anspruch 29, bei der die Einrichtung zum lösbaren Fixieren (10) des Gelenks (6, 7) in verschiedenen Schwenkstellungen eine Schraubverbindung (10) aufweist, welche eine Durchgangslöcher der Kugel oder des Kugelabschnittes (6) und der Kugelschale (7) durchgreifende Schraube hat, wobei mindestens eines der Durchgangslöcher eine die Schraube mit Abstand umgebende Begrenzung (9) aufweist.

31. Orthesen nach Anspruch 30, bei der die Kugel oder der Kugelabschnitt (6) oder die Kugelschale (7) drehbar und in bestimmten Drehstellungen lösbar fixierbar mit dem Abschnitt der Verbindungsstruktur (1) zwischen den Gelenken (6, 7) verbunden ist.

32. Orthese nach Anspruch 31, bei der mindestens ein Gelenk (6, 7) eine Einrichtung zum Begrenzen des Schwenkwinkels in mindestens einer Schwenkebene aufweist.

33. Orthese nach einem der Ansprüche 1 bis 32, bei der die Verbindungsstruktur (1) zwei parallele Stangen (2, 3) und mindestens eine Klemmvorrichtung (5) und/oder Schraubvorrichtung zum lösbaren Fixieren der parallelen Stangen (2, 3) in verschiedenen Stellungen zueinander aufweist.

## Claims

1. Orthotic appliance [corrective limb fitment] for treatment of club-foot, which features fastening devices (24, 27, 28) for securing the patient's foot, and a connecting structure (1), which links the fastening devices (24, 27, 28) and holds them in specific positions in relation to each other, and which is at least partially elastic, **characterized in that** fact that at least one elastic section (37) is arranged on the side facing away from a fastening device (24) on at least one joint (6, 7), between this joint (6, 7) and a further section of the connecting structure (I), and/or that at least one elastic section (21, 34) is arranged between at least one joint (6, 7) and a fastening device (24, 27 28).

2. Orthotic appliance according to claim 1, comprising fastening devices (27, 28) for releasably fastening the patient's shoe.

3. Orthotic appliance according to claim 2, in which the fastening devices comprise means (27, 28) for releasably fixing shoes of the patient onto plates (24).

4. Orthotic appliance according to claim 3, in which the means of releasable fixing (27, 28) feature devices for the shape-fitting connection of the shoes with the plates and/or for the quick release (27, 28) of the shoes from the plates.

5. Orthosis according to one of claims 1 to 4 in which the connecting structure (I) features at least one joint (6, 7) and a device for detachable fixing (10) of the joint at various pivoting positions.

6. Orthosis according to Claim 5, in which the connecting structure features two joints (6, 7), devices for detachable fixing (10) of both joints at various pivoting positions and a section (2, 3) of the connecting structure (1) between both joints.

7. Orthosis according to Claim 6, in which both joints (6, 7) each feature a joint part (7) that is connected to a fixing device (24, 27, 28).

8. Orthosis according to one of claims I to 7, in which the connecting structure (1) is elastic overall.

9. Orthosis according to one of Claims 1 to 7 in which the connecting structure (1) features at least one elastic section (21, 34).

10. Orthosis in accordance with Claims 5 to 9 in which the connecting structure (1) features at least one elastic section (21, 34) outside of at least one joint (6, 7).

11. Orthosi according to claim 9 or 10, in which at least one elastic section (21, 34) is a section of the connecting structure arranged between two joints (6,7).

12. Orthosis according to one of the claims 1 to 11, in which the additional section (2, 3) of the connecting structure is rigid.

13. Orthosis according to one of the claims 1 to 12, in which a rigid additional section (2, 3) of the connecting structure (1) is arranged between two joints (6, 7) and an elastic section (36) is arranged between each joint (6, 7) and the rigid additional section (2, 3) of the connecting structure (1).

14. Orthosis according to one of the claims 1 to 13, in which a rigid section of the connecting structure (1) is arranged between two joints (6, 7) and there is an elastic section between each joint (6, 7) and a fastening device (24).

15. Orthosis according to one of the claims 9 to 14, in which the elastic section (21) is cylindrical.

16. Orthosis according to one of the claims 1 to 15, in which the elastic section (34) forms an integral element of the connecting structure.

17. Orthosis according to one of the claims 1 to 16, in which the elastic section is an elastic component (21).

18. Orthosis according to claim 17, in which the elastic component (21) comprises of a coil spring and/or an elastomer component.

19. Orthosis according to claim 17 or 18 in which the elastic component (21) features threaded bolts and/or threaded bushes in order to connect to adjacent sections of the connecting structure and/or the fixing device.

20. Orthosis according to claim 19 in which the threaded bolts and/or the threaded bushes are fixed into the elastomer component (21).

21. Orthosis according to one of claims 9 to 20 in which the elastic section (21) is equipped with a device (17) for limiting deformability of the elastic section.

22. Orthosis according to claim 21 in which the device (17) for limiting deformability comprises a surrounding border at least partially enclosing the elastic section (21).

23. Orthosis according to claim 22 in which the border (17) covers the elastic section (21) at various perimeter positions to a varied extent.

24. Orthosis according to claims 22 or 23 in which the border (17) features a low point and the diametrically opposite side features a high point with a gradually increasing progression from the low point to the high point inbetween.

25. Orthosis according to one of the Claims 22 to 24, in which the enclosing part (17) is cylindrical.

26. Orthosis according to claims 22 to 25, in which at least one joint (6, 7) features a joint element (7) with an enclosing part (17), which at least partially takes in an elastic section (21).

27. Orthosis according to one of claims 6 to 26, in which at least one joint (6, 7) is a pivoting joint.

28. Orthosis according to claim 27, in which the pivoting joint (6, 7) features two pivoting levels being perpendicular to each other.

29. Orthosis according to one of claims 6 to 28, in which at least one joint (6, 7) features a ball or section of a ball, on which a ball-socket (7) is pivotally arranged.

30. Orthosis according to claim 29, in which the device (10) for releasably fixating the joint (6, 7) features a screw-connection (10) in various pivoting positions, the screw-connection having a screw which passes through holes in the ball or section of ball (6) and in the ball-socket (7), and where at least one of the holes features a barrier (9) surrounding the screw at a certain distance therefrom.

31. Orthosis according to claim 30, in which the ball or the section of the ball (6) or the ball-socket (7) is rotatably, and in certain rotary positions detachably and fixably connected to the section of the connecting structure (1) between the joints (6,7).

32. Orthosis in accordance with Claim 31 in which at least one joint (6, 7) features a device for restricting the pivoting angle in at least pivoting plane.

33. Orthosis in accordance with one of Claims 1 to 32 in which the connecting structure (1) features two parallel rods (2, 3) and at least one clamping device (5) and/or screwing device for detachably fixing of the parallel rods (2, 3) to one another at various positions.

## Revendications

1. Orthèse pour le traitement du pied bot possédant des systèmes d'attache (24, 27, 28) pour attacher les pieds d'un patient, et une structure de raccordement (1) qui relie les systèmes d'attache et les maintient dans des positions définies les unes par rapport aux autres, et qui est au moins partiellement élastique, **caractérisée en ce qu'**au moins une section élastique (37) est disposée sur le côté opposé d'au moins un joint (6, 7) d'un système d'attache (24), entre ce joint (6, 7) et une autre section de la structure de raccordement (1) et/ou qu'au moins une section élastique (21, 34) est disposée entre au moins un joint (6, 7) et un système d'attache (24, 27, 28).

2. Orthèse, selon la revendication 1, qui comporte des systèmes d'attache (27, 28) pour fixation détachable des chaussures du patient.

3. Orthèse, selon la revendication 2, pour laquelle les systèmes d'attache comportent des moyens pour une fixation détachable (27, 28) des chaussures du patient à des plaques (24)

4. Orthèse, selon la revendication 3, pour laquelle les moyens pour une fixation détachable (27, 28) comportent des dispositifs permettant un raccordement parfait et/ou le refroidissement (27, 28) des chaussures sur les plaques (24).

5. Orthèse, selon une des revendications de 1 à 4, pour laquelle la structure de raccordement comporte au moins un joint (6, 7) et un dispositif permettant une fixation détachable (10) du joint dans les différentes positions de pivotement.

6. Orthèse selon la revendication 5, pour laquelle la structure de raccordement comporte deux joints (6, 7), des dispositifs permettant une fixation détachable (10) des deux joints dans les différentes positions de pivotement et une section rigide (2, 3) de la structure de raccordement (1) entre les deux joints.

7. Orthèse selon la revendication 6, pour laquelle les deux joints (6, 7) comportent chacun une partie articulée (7), qui est relié à un dispositif de fixation (24, 27, 28).

8. Orthèse, selon une des revendications de 1 à 7, pour laquelle la structure de raccordement (1) est élastique dans l'ensemble.

9. Orthèse, selon une des revendications de 1 à 7, pour laquelle la structure de raccordement (1) comporte au moins une section élastique (21, 34).

10. Orthèse, selon une des revendications de 5 à 9, pour laquelle la structure de raccordement (1) comporte au moins une section élastique (21,34) à l'extérieur d'au moins un joint (6, 7).

11. Orthèse, selon la revendication 9 ou 10, pour laquelle au moins une section élastique (21, 34) est une section de la structure de raccordement (1) disposée entre deux joints (6, 7).

12. Orthèse, selon une des revendications de 1 à 11, pour laquelle la section (2, 3) supplémentaire de la structure de raccordement (1) est rigide.

13. Orthèse, selon une des revendications de 1 à 12, comportant une section rigide (2, 3) supplémentaire entre deux joints (6, 7) de la structure de raccordement (1) ainsi qu'une section élastique (36) entre chaque joint (6, 7) et la section supplémentaire rigide (2, 3) de la structure de raccordement (1).

14. Orthèse, selon une des revendications de 1 à 13, comportant une section rigide de la structure de raccordement (1) tous les deux joints (6, 7) et une section élastique est prévue entre chaque joint (6, 7) et un dispositif de fixation (24).

15. Orthèse, selon une des revendications de 9 à 14, pour laquelle la section élastique (21) est élastique.

16. Orthèse, selon une des revendications de 1 à 15, pour laquelle la section élastique (34) est intégralement formée avec la structure de relation.

17. Orthèse, selon une des revendications de 1 à 16, pour laquelle la section élastique est un élément (21).

18. Orthèse selon la revendication 17, pour laquelle l'élément (21) contient un ressort à boudin et/ou un élément élastomère.

19. Orthèse selon la revendication 17 ou 18, pour laquelle l'élément élastique (21) comporte des goujons filetés et/ou des raccords filetés pour faire la liaison avec les sections avoisinantes de la structure de raccordement et/ou du dispositif de fixation.

20. Orthèse selon la revendication 19, pour laquelle les goujons filetés et/ou les raccords filetés sont ancrés dans l'élément élastomère (21).

21. Orthèse, selon une des revendications de 9 à 20, pour laquelle un dispositif limitant (17) la déformation de la section élastique (21) est affecté à cette dernière.

22. Orthèse selon la revendication 21, pour laquelle le dispositif limitant (17) la déformation comporte une des sections élastiques (21) entourant au moins en partie la bordure.

23. Orthèse selon la revendication 22, pour laquelle la bordure (17) couvre la section élastique (21) à différentes positions circonférentielles dans différentes proportions.

24. Orthèse selon une des revendications 24 ou 25, pour laquelle la bordure (17) comporte un point le plus bas et un point le plus haut sur le coté diamétralement opposé et entre les deux un tracé augmentant progressivement du point le plus bas au point élevé.

25. Orthèse, selon une des revendications de 22 à 24, pour laquelle la bordure (17) est cylindrique.

26. Orthèse selon les revendications 22 à 25, pour laquelle au moins un joint (6, 7) comporte un élément de joint (7) avec une bordure (17), qui accueille au moins partiellement une section élastique (21).

27. Orthèse, selon une des revendications de 6 à 26, pour laquelle au moins un joint (6, 7) est un joint pivotant.

28. Orthèse selon la revendication 27, pour laquelle le joint pivotant (6, 7) comporte deux niveaux pivotant verticalement l'un par rapport à l'autre.

29. Orthèse, selon une des revendications de 6 à 28, pour laquelle au moins un joint (6, 7) comporte une boule ou une section sphérique (6), sur laquelle est disposée une coque sphérique (7) rabattable.

30. Orthèse selon la revendication 29, pour laquelle le dispositif permettant une fixation détachable (10) du joint (6, 7) dans les différentes positions de pivotement comporte une fixation à vis (10), laquelle a une vis entourant les orifices de passage de la boule ou de la section sphérique (6) et de la coque sphérique (7), auquel cas un des orifices de passage comporte une limitation (9) entourant la vis de loin.

31. Orthèse selon la revendication 30, pour laquelle il est possible de tourner la boule ou la section sphérique (6) ou la coque sphérique (7) dans certaines positions rotatives, elle peut ainsi être détachée, elle peut être fixée avec la section de la structure de raccordement (1) et elle est reliée entre les joints (6, 7).

32. Orthèse selon la revendication 31, pour laquelle au moins un joint (6, 7) comporte un dispositif limitant l'angle d'orientation dans au moins un plan de basculement.

33. Orthèse selon une des revendications de 1 à 32, pour laquelle la structure de raccordement (1) comporte deux barres parallèles (2, 3) et au moins un dispositif de serrage (5) et/ou un dispositif de vissage pour fixation détachables des barres parallèles (2, 3) dans différentes positions les unes par rapport aux autres.
